# NEW EUROPEAN PATENT SPECIFICATION

(11) **EP 1 137 382 B2**
(45) Date of publication and mention of the opposition decision: **22.08.2012**
(45) Mention of the grant of the patent: 25.06.2003
(21) Application number: 99971299.5
(22) Date of filing: 27.10.1999
(51) Int. Cl.: A61F 13/15, B32B 3/26, B32B 7/12

(54) **TOPSHEET SYSTEM FOR ABSORBENT ARTICLES**
DECKSCHICHTSYSTEM FÜR ABSORBIERENDE ARTIKEL
SYSTEME A COUCHE SUPERIEURE POUR ARTICLES ABSORBANTS

(30) Priority: 30.10.1998 US 183289
(43) Date of publication of application: 04.10.2001
(73) Proprietor: THE PROCTER & GAMBLE COMPANY, Cincinnati, OH 45202 (US)
(72) Inventor: HASSE, Margaret, Henderson, Cincinnati, OH 45215 (US)
(74) Representative: Briatore, Andrea
(86) International application number: PCT/US1999/025268
(87) International publication number: WO 2000/025714

(56) References cited:
- EP-A- 0 545 423
- WO-A-97/09017
- WO-A2-95//03021
- US-A- 4 614 679
- US-A- 4 629 643
- US-A- 4 917 697
- US-A- 5 281 208
- US-A- 5 342 469
- US-A- 5 383 870
- US-A- 5 591 149

## Description

The present invention relates to topsheet systems for disposable absorbent articles, such as catamenials, sanitary pads, and other feminine hygiene products.

### BACKGROUND OF THE INVENTION

It has long been known in the field of disposable absorbent articles that it is extremely desirable to construct absorptive devices, such as disposable diapers, sanitary napkins, incontinent briefs, bandages, wound dressings, and the like, that are highly effective in receiving and containing urine, menses, and other body exudates. Accordingly, it is generally desirable to promote rapid fluid transfer in a direction away from the wearer and into a retentive structure, while resisting fluid transfer in the reverse direction either toward the wearer or toward external garments or surfaces.

One viable prior art solution to the former problem is to utilize a covering or topsheet on the exposed, wearer-contacting surface that comprises a web of formed, apertured, thermoplastic film. Commonly assigned U.S. Pat. No. 4,342,314, issued to Radel et al. on August 3, 1982, the disclosure of which is hereby incorporated herein by reference, discloses a representative formed film of this variety. Such webs are macroscopically-expanded to form three-dimensional structures capable of capillary fluid transport. They are used to conduct fluid away from one surface (wearer-facing) into and through the web via three-dimensional capillaries formed into the material to a second surface (garment-facing), and then into the underlying absorbent structure. The first surface is continuous, and is in a spaced apart, generally parallel relationship to the second surface, which is discontinuous.

To address consumer concerns with regard to plastic-like appearance and feel, webs of the variety taught by Radel have been developed which include an interconnected structure of fiber-like appearance in the interest of generating a more cloth-like, aesthetically-pleasing appearance. In addition, apertured, formed film webs have been developed which further include microscopic surface texturing (microtexture) and/or microscopic apertures (microapertures) to further enhance the visual and tactile impression of such webs. Representative film webs of this variety are disclosed in commonly assigned U.S. Pat. Nos. 4,463,045, issued to Ahr et al. on July 31, 1984, and 4,629,643, issued December 16, 1986 to Curro et al. Representative of the art on absorbent articles with apertured layer joined to adjacent layer include WO 97/09017, US 5 591 149, US 4 614 679 and US 5 383 870.

Three-dimensional, formed-film webs of the foregoing varieties are often used in combination with secondary topsheets, absorbent cores, or other subjacent components to form a topsheet system. As discussed above, the second (garment-facing) surface of typical formed-film three-dimensional webs is in a spaced apart relationship to the first (body-facing) surface. Therefore, the second surface can be joined to the subjacent layer, such as by adhesive bonding, such that the first surface is in a spaced apart relationship to the subjacent layer as well. Due to the columnar aspect of the walls of the three-dimensional capillaries, the first surface often remains in a spaced apart relationship to the subjacent layer. This spaced apart relationship promotes a dry body-facing first surface and increases the masking properties of the web, that is, the ability of a web used as a topsheet on a disposable absorbent article to mask the absorbed fluid (e.g., blood) from view after it passes through the web.

While such topsheet systems (i.e., formed film adhered to a subjacent layer) can be effective in transporting fluid, their effectiveness can be limited by the permanent collapse of the inherent columnar, three-dimensional structure of the formed film web during manufacture or use. Once collapsed, formerly un-adhered portions of the web (i.e., portions not on the second surface of the web) may become adhered, the web thereby being unable to recover to its previously un-compressed three-dimensional, stand-off configuration. When this occurs the three-dimensional capillaries no longer retain their structure, and may even become completely blocked.

This unintended permanent collapse of the three dimensional structure is due to the fact that the adhesive used to adhere the second surface of the capillary web to the subjacent structure remains tacky long after application, so that once sufficiently compressed, other portions of the capillary web become adhered to the subjacent layer. Sufficient compression may occur during manufacture or in normal use by the wearer of a disposable article using such a topsheet system. Thereafter the capillaries do not function effectively, resulting in decreased fluid handling capability of the topsheet system (resulting in a wetter first surface), and poor masking properties.

Various approaches in the art have attempted to address these issues, including the elimination of any adhesive between the capillary web and the subjacent layer, and the use of alternative joining methods, such as ultrasonic bonding. However, most topsheet systems require adhesive to ensure sufficient contact between the topsheet components, which promotes much more effective fluid transport. Moreover, non-adhesive methods, such as ultrasonic bonding, introduce added processing costs and complexity, as well as imposing material limitations on the component members of a topsheet system.

Accordingly, it would be desirable to provide a topsheet system effective in transporting fluid away from a body-facing surface during use, including resistance to permanent collapse of three-dimensional capillary structures under pressure.

More particularly, it would be desirable to prevent permanent collapse of the three-dimensional capillary structures in a topsheet system during manufacture of the topsheet system, or during use of an article utilizing such a system.

### SUMMARY OF THE INVENTION

A topsheet system is disclosed, the topsheet system comprising a primary topsheet having a first surface and a second surface generally parallel to and spaced apart from the first surface. The primary topsheet comprises a plurality of fluid passageways extending between the first surface and the second surface to place the first surface and the second surface in fluid communication with one another. The topsheet system also comprises a secondary layer subjacent to and bonded to the second surface of the primary topsheet at predetermined portions by a hot melt adhesive. The adhesive has a residual tack of less than 16 grams per inch peel force, thus it has essentially no residual tack, such that after joining, subsequent contact between the primary topsheet and the secondary layer at areas other than the predetermined portions prevents bonds of measurable strength between the primary topsheet and the secondary layer. An absorbent article utilizing a topsheet system of the present invention is also disclosed.

### BRIEF DESCRIPTION OF THE DRAWINGS

While the specification concludes with claims particularly pointing out and distinctly claiming the subject matter of the present invention, it is believed that the present invention will be better understood from the following description taken in conjunction with the accompanying drawings in which like reference numerals identify identical elements and wherein:
FIG. 1 is an enlarged, partially segmented, perspective illustration of a prior art polymeric web of a type generally disclosed in commonly assigned U.S. Pat. No. 4,342,314 to Radel et al.;
FIG. 2 is an enlarged, partially segmented, perspective illustration of a prior art polymeric web of a type generally disclosed in commonly assigned U.S. Pat. No. 4,629,643 to Curro et al.;
FIG. 3 is a plan view of aperture shapes projected in the plane of the first surface of an alternative compression resistant web of the present invention;
FIG. 4 is a further enlarged, partial view of a web of the type generally shown in FIG. 2, but illustrating in greater detail the web construction;
FIG. 5 is a cross-sectional illustration of an uncompressed topsheet system, representative of a topsheet system of the present invention;
FIG. 6 is a cross-sectional illustration of a compressed topsheet system showing the problem of permanent collapse of topsheet systems employing existing adhesives;
FIG. 7 is a cross-sectional illustration of an alternative topsheet system, representative of a topsheet system of the present invention;
FIG. 8 is a top plan view of a sanitary napkin with portions cut way to more clearly show the construction of the sanitary napkin;
FIG. 9 is a cross-sectional view of the sanitary napkin of FIG. 8 taken along line 9-9;

### DETAILED DESCRIPTION OF THE PRESENT INVENTION

The topsheet system of the present invention comprises three basic components: a primary topsheet; a secondary layer subjacent to the primary topsheet; and an adhesive layer that bonds the primary topsheet to the secondary layer. The primary topsheet may be a fluid pervious web, such as a nonwoven web, and is preferably a formed film web, such as a three-dimensional, macroscopically-expanded, apertured web. The secondary layer may be a secondary topsheet, such as a nonwoven web, or it may be an absorbent core component of an absorbent article.

In general, the primary topsheet has a first, body-facing surface, and a second, garment-facing surface generally parallel to and spaced apart from the first surface. In other words, the primary topsheet has measurable Z-direction caliper, and is fluid pervious such that the first surface and second surfaces are in fluid communication with one another. In general, bonding between the primary topsheet and the secondary layer occurs at predetermined portions of the second surface of the primary topsheet. The predetermined portions may comprise essentially all of the second surface of the primary topsheet, or some fraction thereof. For example, adhesive applied in a low coverage spiral pattern would only adhere some fraction of the total second surface of the primary topsheet.

The primary topsheet can comprise fluid pervious woven materials, nonwoven materials, paper webs, foams, battings, and the like such as are known in the art. Nonwoven webs can have fibers or filaments distributed randomly as in "air-laying" or certain "wet-laying" processes, or with a degree of orientation, as in certain "wet-laying" and "carding" processes. The fibers or filaments of the nonwoven can be natural, or of natural origin (e.g. cellulosic fibers such as wood pulp fibers, cotton linters, rayon, and bagasse fibers) or synthetic (e.g. polyolefins, polyamides or polyesters), or blends thereof.

The adhesive of the present invention has essentially no residual tack, such that after joining the primary topsheet experiences little or no permanent collapse under pressure. By using an adhesive having essentially no residual tack, previously un-adhered portions of the primary topsheet do not become adhered to the secondary topsheet upon deformation of the three-dimensional structure. For example, if a nonwoven web is used as the primary topsheet, the second, garment-facing, surface may be adhered to the secondary layer, leaving the first, body-facing, surface in a spaced, unadhered relationship to the second surface. Upon subsequent compression of the topsheet system, the fibers of the first surface may contact the secondary layer, but do not become adhered to the secondary layer. Therefore, permanent collapse of the three-dimensional character of the topsheet system is avoided.

As used herein, the term "tack" refers to the property of an adhesive that enables it to form a bond of measurable strength, for example the bond between the primary topsheet and secondary layer, such that predetermined portions of the primary topsheet can be bonded under pressure.

As used herein, "residual tack" refers to the property of an adhesive to form a measurable bond at some time subsequent to application of the adhesive. The topsheet system of the present invention utilizes a hot melt adhesive that exhibits a residual tack of less than 16 grams per inch peel force, thus it has essentially no residual tack several days after application, and more preferably, exhibits essentially no residual tack shortly after application.

By "essentially no" residual tack is meant that, after initial bonding of predetermined portions of the primary topsheet, subsequent pressures of manufacturing or use result in little or no bond of measurable strength between previously unbonded portions of the primary topsheet and secondary layer, specifically less than 16 grams per inch peel force.

By "shortly after" application is meant less than 10 minutes after application, preferably less than 1 minute after application, more preferably less than 10 seconds after application, and most preferably less than 1 second after application.

The topsheet system of the present invention, therefore, utilizes an adhesive with essentially no residual tack, thereby preventing previously unadhered portions of the primary topsheet from becoming subsequently adhered to the secondary layer when compressed during later manufacturing steps, or during use. In other words, the topsheet system of the present invention resists permanent collapse of the three-dimensional nature of the primary topsheet upon application of pressure. The invention is described in detail below with respect to the Figures, in which like reference numerals identify identical elements.

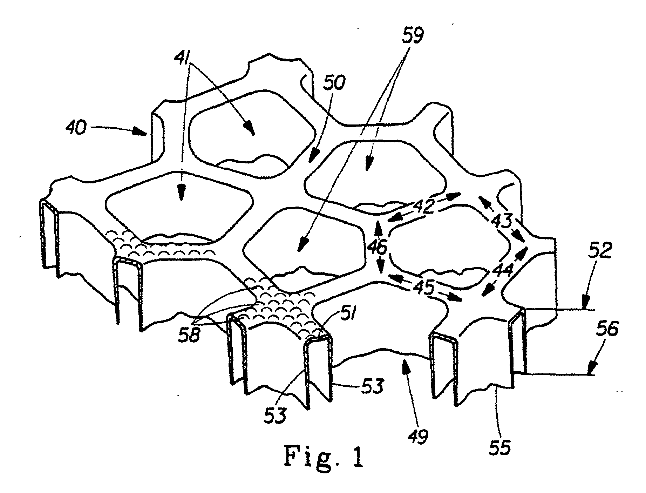

FIG. 1 is an enlarged, partially segmented, perspective illustration of a prior art macroscopically-expanded, three-dimensional, fiber-like, fluid pervious polymeric web **40** which has been found highly suitable for use as a topsheet in disposable absorbent articles, such as diapers and sanitary napkins, as well as in topsheet systems of the present invention. The prior art web is generally in accordance with the teachings of commonly assigned U.S. Pat. No. 4,342,314 issued to Radel et al. on Aug. 3, 1982, which is hereby incorporated herein by reference. The fluid pervious web **40** exhibits a multiplicity of apertures, e.g., apertures **41,** which are formed by a multiplicity of interconnected fiber-like elements, e.g., fiber-like elements **42, 43, 44, 45,** and **46** interconnected to one another in the first surface **50** of the web. Each fiber-like element comprises a base portion, e.g., base portion **51,** located in plane **52** of the first surface **50.** Each base portion has a sidewall portion, e.g., sidewall portion **53,** attached to each edge thereof. The sidewall portions extend generally in the direction of the second surface **55** of the web. The intersecting sidewall portions of the fiber-like elements are interconnected to one another intermediate the first and second surfaces of the web, and terminate substantially concurrently with one another in the plane **56** of the second surface **55.**

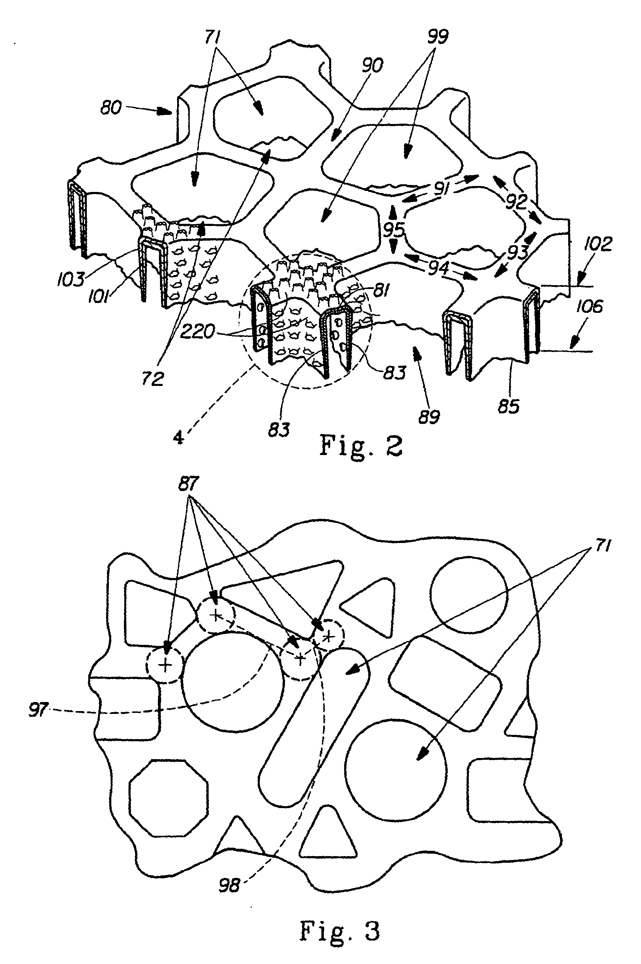

In a preferred embodiment, the base portion **51** includes a microscopic pattern of surface aberrations **58,** which can be generally in accordance with the teachings of U.S. Pat. No. 4,463,045, issued to Ahr et al. on July 31, 1984, the disclosure of which is hereby incorporated herein by reference. When present according to the teachings of Ahr et al., the microscopic pattern of surface aberrations **58** provides a substantially non-glossy visible surface when the web is struck by incident light rays. In a more preferred embodiment, surface aberrations **58** can be hydroformed microapertures in accordance with the teachings of the aforementioned '643, issued to Curro et al., as discussed below with reference to FIG. 2.

In an alternative embodiment the prior web may include a multiplicity of much smaller capillary networks (not shown) in the first surface **50** of the web, as taught by U.S. Pat. No. 4,637,819 to Ouellette et al. issued Jan. 20, 1987 and hereby incorporated herein by reference. It is believed that the additional porosity afforded by the smaller fluid-handling capillary networks may allow the web of the present invention to function more efficiently when used as a topsheet of a disposable absorbent article.

As utilized herein, the term "interconnecting members" refers to some or all of the elements of the web, portions of which serve to define the primary apertures by a continuous network. Representative interconnecting members include, but are not limited to, the fiber-like elements of the aforementioned '314 Radel et al. patent and commonly assigned U.S. Patent No. 5,514,105 to Goodman, Jr., et al. issued on May 7, 1996 and hereby incorporated herein by reference. As can be appreciated from the following description and drawings, the interconnecting elements are inherently continuous, with contiguous interconnecting elements blending into one another in mutually-adjoining transition portions.

Individual interconnecting members can best be generally described, with reference to FIG. 1, as those portions of web **40** disposed between any two adjacent primary apertures, originating in the first, body-facing surface **50** and extending to the second, garment-facing surface **55.** On the first surface of the web the interconnecting members collectively form a continuous network, or pattern, the continuous network of interconnecting members defining the primary apertures, (for example, interconnecting members **42, 43, 44, 45,** and **46)** and on the second surface of the web the interconnecting sidewalls of the interconnecting members collectively form a discontinuous pattern of secondary apertures in the second surface of the topsheet. As taught in the aforementioned '105 patent to Goodman, Jr., et al., it may be desirable to have multiple secondary apertures for any one primary aperture.

As utilized herein, the term "continuous", when used to describe the first surface of the web, refers to the uninterrupted character of the first surface, generally in the plane of the first surface. Thus, any point on the first surface can be reached from any and every other point on the first surface without substantially leaving the first surface in the plane of the first surface. Likewise, as utilized herein, the term "discontinuous," when used to describe the second surface of the web, refers to the interrupted character of the second surface, generally in the plane of the second surface. Thus, any point on the second surface cannot be reached from every other point on the second surface without substantially leaving the second surface in the plane of the second surface.

In general, as utilized herein the term "macroscopic" is used to refer to structural features or elements which are readily visible to a normal human eye when the perpendicular distance between the viewer's eye and the plane of the web is about 12 inches. Conversely, the term "microscopic" is utilized to refer to structural features or elements which are not readily visible to a normal human eye when the perpendicular distance between the viewer's eye and the plane of the web is about 12 inches.

As utilized herein, the term "macroscopically-expanded", when used to describe three-dimensional webs, ribbons and films, refers to webs, ribbons and films which have been caused to conform to the surface of a three-dimensional forming structure so that both surfaces thereof exhibit the three-dimensional pattern of the forming structure. Such macroscopically-expanded webs, ribbons and films are typically caused to conform to the surface of the forming structures by embossing (i.e., when the forming structure exhibits a pattern comprised primarily of male projections), by debossing (i.e., when the forming structure exhibits a pattern comprised primarily of female capillary networks), or by extrusion of a resinous melt onto the surface of a forming structure of either type.

By way of contrast, the term "planar" when utilized herein to describe plastic webs, ribbons and films, refers to the overall general condition of the web, ribbon or film when viewed by the naked eye on a macroscopic scale. For example, a non-apertured extruded film or an apertured extruded film that does not exhibit significant macroscopic deformation out of the plane of the film would generally be described as planar.

When macroscopically-expanded, the interconnecting members of the formed film web may be described as channel-like. Their two-dimensional cross-section may also be described as "U-shaped", as in the aforementioned Radel et al. patent, or more generally as "upwardly concave-shaped", as disclosed in the aforementioned Goodman, Jr., et al. patent, which is hereby incorporated herein by reference. "Upwardly concave-shaped" as used herein describes the orientation of the channel-like shape with relation to the surfaces of the web, with the base generally in the first surface, and the legs of the channel extending from the base in the direction of the second surface, and with the channel opening being substantially in the second surface. In general, for a plane extending through the web orthogonal to the plane of the first surface and intersecting any two adjacent primary apertures, the resulting cross-section of an interconnecting member disposed between will exhibit a generally upwardly concave shape that may be substantially U-shaped.

One drawback associated with prior art macroscopically-expanded, three-dimensional webs, as shown in FIG. 1, is that some consumers object to placing polymeric webs comprised of plastic in contact with their skin. Microaperturing the macroscopically-expanded web, generally in accordance with the teachings the aforementioned Curro '643 patent, gives the web increased softness and tactile impression, thereby making the web more clothlike, and less plastic-like. Such microapertures are shown as the surface aberrations **120** of FIG. 2.

FIG. 2 shows a prior art microapertured, macroscopically-expanded, three-dimensional, web embodiment suitable for use as a preferred primary topsheet of the present invention, generally indicated as **80.** The geometrical configuration of the fluid-pervious web **80** is generally similar to that of prior art web **40,** illustrated in FIG. 1, and is generally in accordance with the teachings of the aforementioned '314 Radel et al. and "643 Curro et al. patents. Other suitable formed film patterns and configurations are described in commonly-assigned, U.S. Pat. No. 3,929,135, issued to Thompson on Dec. 30, 1975; U.S. Pat. No. 4,324,246 issued to Mullane, et al. on April 13, 1982; and U.S. Pat. No. 5,006,394 issued to Baird on April 9, 1991. The disclosures of each of these applications and patents are hereby incorporated herein by reference.

A preferred embodiment of an apertured web **80** exhibits a multiplicity of primary apertures, e.g., primary apertures **71,** which are formed in plane **102** of the first surface **90** by a continuous network of interconnecting members, e.g., members **91, 92, 93, 94, 95** interconnected to one another. Primary apertures are also termed "apertures" or "macroapertures" herein, as opposed to "microapertures" present as surface aberrations on web **80,** suitable for use in a topsheet system of the present invention. Macroapertures are the result of macroscopically-expanding a planar web; in general the planar web may or may not be microapertured.

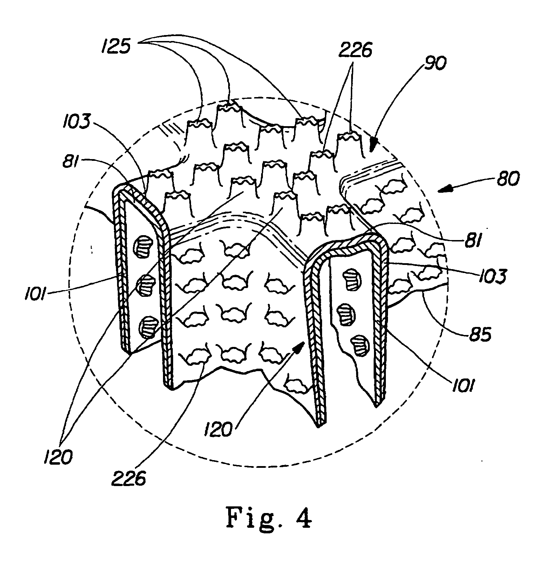

The shape of primary apertures **71** as projected on the plane of the first surface **90** may be in the shape of polygons, e.g., squares, hexagons, etc., in an ordered or random pattern. In a preferred embodiment primary apertures **71** are in the shape of modified ovals, or teardrop shaped, in a staggered pattern. For example, FIG. 4 is a plan view of alternative primary aperture shapes projected in the plane of the first surface of an alternative web believed suitable for use as the primary topsheet of the present invention. While a repeating pattern of uniform shapes is preferred, the shape of primary apertures, e.g., apertures **71,** may be generally circular, polygonal, or mixed, and may be arrayed in an ordered pattern or in a random pattern. Although not shown, it is understood that the projected shape may also be elliptical, tear-drop shaped, or any other shape. Therefore, the present invention is believed to be aperture-shape independent.

The interconnecting elements are inherently continuous, with contiguous interconnecting elements blending into one another in mutually-adjoining transition zones or portions, e.g., transition portions **87,** shown in FIG. 4. In general, transition portions are defined by the largest circle that can be inscribed tangent to any three adjacent apertures. It is understood that for certain patterns of apertures the inscribed circle of the transition portions may be tangent to more than three adjacent apertures. For illustrative purposes, interconnecting members may be thought of as beginning or ending substantially at the centers of the transition portions, such as interconnecting members **97** and **98.** Likewise, the sidewalls of the interconnecting members can be described as interconnecting to sidewalls of contiguous interconnecting members at areas corresponding to points of tangency where the inscribed circle of the transition portion is tangent to an adjoining aperture.

Exclusive of the transition zones, cross-sections transverse to a center line between the beginning and end of interconnecting members are preferably of generally uniform U-shape. However, the transverse cross-section need not be uniform along the entire length of the interconnecting member, and for certain aperture configurations it will not be uniform along most of its length. In particular, in transition zones or portions **87**, interconnecting members blend into contiguous interconnecting members and transverse cross-sections in the transition zones or portions may exhibit substantially non-uniform U-shapes, or no discernible U-shape. Further discussion of transition zones and cross-section shapes may be learned from commonly-assigned, copending U.S. Pat. Application Ser. No. 08/816,106, entitled Tear Resistant Porous Extensible Web, filed in the names of Curro, et al., which is hereby incorporated herein by reference.

FIG. 4 is an enlarged, partially segmented, perspective illustration of a segment of a prior art microapertured polymeric web **80** of FIG. 2. As shown, rupturing of the surface aberrations **120** results in the formation of volcano-shaped apertures **125** having relatively thin, irregularly shaped petals about its periphery. Because the microapertures are formed prior to the macroscopic expansion of the web, the microapertures occur on the first surface of the web, as well as on the sidewalls of the interconnecting members. Microapertures **125** can be formed by a hydroformation process in which a high pressure liquid jet is preferably utilized to force the web to conform to a woven wire support member. Because of the greater driving force applied by the liquid jet, those portions of the web which coincide with the interstices formed by the intersecting filaments in the woven wire support member are ruptured to form tiny apertures, i.e., microapertures **125** at points which coincide substantially with the maximum amplitude of each surface aberration **120.**

It has been found that a microapertured, macroscopically-expanded web provides greatly enhanced softness, but can be more susceptible to collapse under pressure. Without being bound by theory, it appears that the increased susceptibility to collapse under pressure is due to the decreased compression resistance of the microapertured web. Due to the plurality of microaperture openings, which include openings in the sidewalls of the interconnecting members, microapertured webs may not have the structural rigidity to withstand normal use pressure, and retain or recover a certain standoff, i.e., a certain distance between the first surface and the second surface of the web.

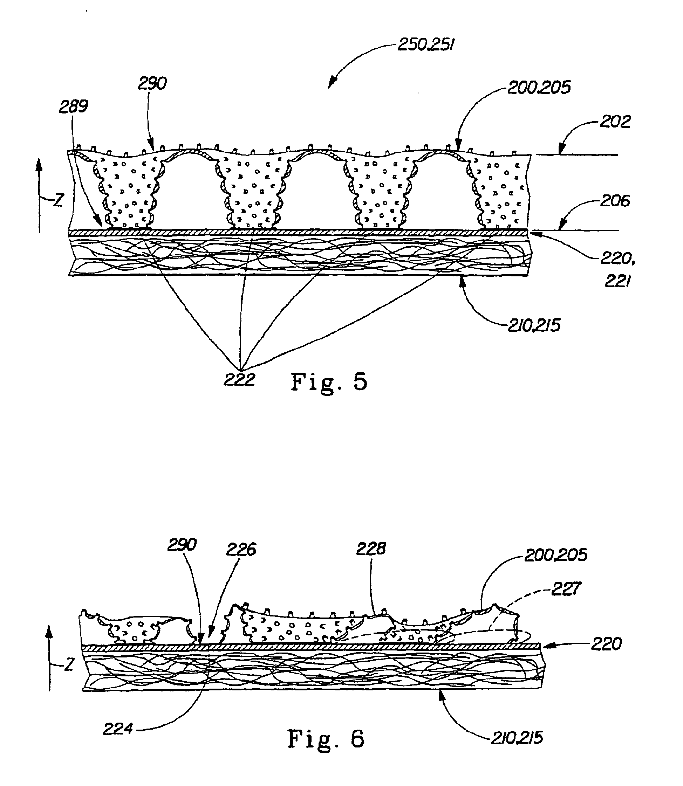

In certain topsheet systems for disposable articles the topsheet is adhered to underlying layers, such as secondary topsheets, absorbent core layers, and the like. For example, FIG. 5 shows a topsheet system **250** that can be a topsheet system of the present invention **251,** depending on the adhesive used, as discussed below. Primary topsheet **200** is bonded at predetermined portions **222** to secondary layer **210.** Primary topsheet **200** can be an apertured, formed film web **205,** as shown in FIG. 5, adhered by adhesive layer **220** to a secondary, subjacent layer **210,** shown as a fluid pervious nonwoven web layer **215.** Primary topsheet **200** can be adhered to subjacent layer **210** at predetermined portions **222.** Adhesive layer **220** may be applied prior to bonding as a continuous layer, or it may be applied as discontinuous stripes, spirals, spots, and the like. However, in general, once it is applied, some amount of adhesive remains in unbonded areas **224.**

In existing topsheet systems **250,** the adhesive **220** in unbonded regions **224** can remain tacky long after bonding of the primary topsheet layer to the subjacent layer. Therefore, one drawback to having a collapsible, i.e., deformable in the Z-direction, web as a primary topsheet in such topsheet systems is its tendency to compress during use, adhere to previously unadhered areas **224,** and remain collapsed after pressure is released.

As shown in FIG. 6, once compressed, certain areas of the first surface of the apertured web may be compressed relatively parallel to the Z-direction, as indicated by the compressed area **226,** which has collapsed and become adhered in the previously unadhered portion **224.** Likewise, portions of the first surface of the primary topsheet may be compressed laterally, i.e., substantially orthogonal to the Z-direction, as shown by laterally compressed areas **228.** In extreme cases, the primary topsheet may become effectively completely flattened, as shown by dotted outline **227.** If portions of the primary topsheet become adhered in this position, the apertures become effectively blocked, the primary topsheet may not transport fluid effectively, and the masking properties of a three-dimensional web are minimized. Masking reflects the visual cleanliness of the surface after fluid pass-through, further characterized by the degree of coloration remaining (with a colored fluid) as well as the size or extent of the discolored region.

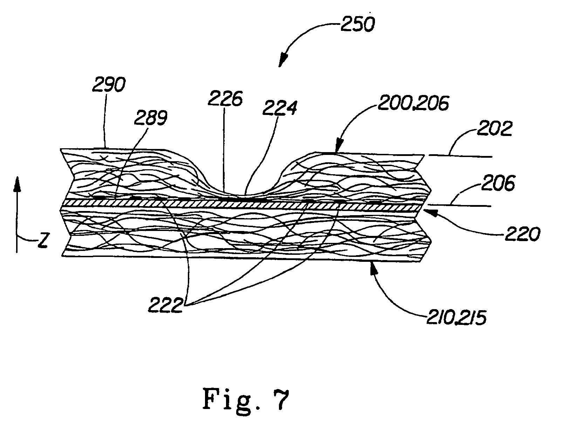

Likewise, if a nonwoven web **206** is utilized as a primary topsheet **200** in a topsheet system **250,** as shown in FIG. 7, deformed portions of the primary topsheet can become permanently compressed. As shown, nonwoven primary topsheet **206** can have a first surface **290** generally in the plane of the first surface **202,** and a second surface **289** generally in the plane of the second surface **206.** When initially joined together, predetermined portions **222** of the second surface of nonwoven web primary topsheet **206** are bonded by adhesive. Thereafter, if the adhesive retains residual tack, the first surface of the primary topsheet **206** can be compressed relatively parallel to the Z-direction, as indicated by the compressed area **226,** and can become adhered in the previously unadhered portion **224.**

The problems associated with providing soft nonwoven webs or microapertured, formed-film webs for use as topsheets in absorbent articles are overcome with a topsheet system **251** of the present invention. The topsheet system of the present invention is structurally similar to the topsheet system shown in FIGs. 5 and 7, with a preferred embodiment shown in FIG. 5. The topsheet system **251** of the present invention is illustrated with reference to FIG. 5, with the distinction over the description above being that the adhesive layer **220** comprises an adhesive **221** having essentially no residual tack in place of prior art adhesives, including prior art hot melt adhesives.

The topsheet system **251** of the present invention comprises a primary topsheet **200,** preferably a microapertured web **205,** and a secondary subjacent layer **210,** preferably a nonwoven web **215.** The primary topsheet of the present invention **200** can have a first surface **290,** generally in the plane of the first surface **202,** and a second surface **289** generally in the plane of the second surface **206.**

By utilizing an adhesive having essentially no residual tack to bond the primary topsheet to the secondary subjacent layer, the technical problem of permanent collapse upon compression is resolved. For example, when deformed as shown in FIG. 6, the primary topsheet of the present invention does not become bonded at previously unbonded regions 224. Once the compression load is removed, the primary topsheet can then return to its original position, thereby restoring the three-dimensional nature of the primary topsheet. Permanent compression is avoided, and the plane of the first surface 202 can remain in a generally parallel spaced apart relationship to the plane of the second surface 206, even after repeated compression cycles.

Therefore, when used as a topsheet on an absorbent article, the topsheet system of the present invention exhibits improved fluid transport properties during use, including fluid acquisition, reduced rewet, and improved masking. For example, preventing or at least minimizing permanent compression greatly increases the masking effectiveness of the topsheet by allowing the primary topsheet to recover a certain degree of standoff, or distance from the first (body facing) surface **290** of the primary topsheet to the second (garment facing) surface **289.**

Some suitable adhesives having essentially no residual tack are shown in Table 1, along with a widely used prior art adhesive. Table 1 shows the peel force in grams as determined using the Test Method below. The peel force represents the force to peel apart two substrates previously bonded and put under uniform pressure to simulate pressure during product use. In particular, two pressures were tested, 0.375 psi, and 1.25 psi, which have been determined to simulate a wide range of pressure typically applied to a sanitary pad during use. For each pressure, each adhesive shown was tested on three combinations of primary topsheets and secondary layers. The primary and secondary layer substrates are described as follows:
AW:NW: Stands for Apertured Web:Nonwoven. A primary topsheet comprised of a three-dimensional, apertured web, similar to the web shown in FIG. 2 was bonded to a secondary topsheet comprised of a spunbond polypropylene nonwoven. Suitable spunbond nonwovens are available from Fiberweb, for example Fiberweb P9. This combination of materials (i.e., a three-dimensional, apertured primary topsheet bonded to a nonwoven secondary layer) represents a preferred topsheet system of the present invention.
PE:PE: Two identical, nonapertured, planar polyethylene sheets having a caliper of about 1 mil were bonded together, and tested for comparison purposes.
Cotton:PE: As further comparison to a preferred topsheet system, cotton batting was bonded to a nonapertured, planar polyethylene sheet.

Adhesives tested are shown in the far left column of Table 1. The first adhesive, Ato-Findley H2031 is an adhesive used on some current topsheet systems. H2031 is considered to be unacceptable as an adhesive for the present invention, that is, it has relatively high residual tack (about 75 grams), which has been determined to produce permanent collapse of the primary topsheet upon deformation under pressure. Ato-Findley adhesives are available from Findlay Adhesives of Wauwatosa, Wisconsin.

The remaining adhesives shown in Table 1 are suitable for use as the adhesive in a topsheet system of the present invention, particularly for preferred embodiments utilizing an apertured formed film web primary topsheet and a nonwoven web secondary layer. In a preferred embodiment, adhesives having essentially no residual tack at the pressures shown on a AW:NW combination can be used. Such adhesives include National Starch 34-5643S polyolefin hot melt adhesive, available from National Starch of Bridgewater, NJ, and H.B. Fuller HL-1628-B block copolymer adhesive, available from Fuller of St. Paul, MN. Less desirable, but suitable for the topsheet system of the present invention include Ato-Findley HX4111 and H2600, both available from the Ato-Findley Co., and H.B. Fuller HL-1472-XZP, available from the H.B. Fuller Co. Each value shown in Table 1 can be normalized to units of grams/inch simply by dividing the value shown by 2.5 inches (the width of the test samples as set forth in the Test Method below). For example, a peel force of 38 grams for a strip 2.5 inches wide can be normalized to about 15.2 grams/linear inch, or grams/inch. In general, adhesives having a normalized peel force less than about 16 grams/inch are suitable for use in a topsheet system of the present invention. Adhesives having less than about 16 grams/inch normalized peel force are considered to have essentially no residual tack.

**Table 1 -**

| Peel Force Data | | | | | | |
|---|---|---|---|---|---|---|
| **Adhesive** | **Average Peel Force (grams)** | | | | | |
| | **@0.375 psi** | | | **@1.25 psi** | | |
| | **AW:NW** | **PE:PE** | **Cotton:PE** | **AW:NW** | **PE:PE** | **Cotton:PE** |
| Ato-Findley H2031 | 75±6 | 183±35 | 78±5 | 134±15 | 280±38 | 161±24 |
| Ato-Findley HX4111 | 38±5 | 143±19 | 53±10 | 53±7 | 276±38 | 92±15 |
| Fuller HL-1472-X ZP | 14±2 | 9±2 | 59±5 | 22±4 | 19±7 | 144±18 |
| Ato-Findley H2600 | 10±1 | 4±1 | 38±7 | 17±3 | 16±6 | 148±27 |
| National Starch 34-5643S | 0 | 0 | 2±1 | 0 | 0 | 6±2 |
| Fuller HL-1628-B | 0 | 0 | 4±1 | 0 | 0 | 12±2 |

Suitable adhesives may be applied utilizing techniques conventional in the art for such applications, including a uniform continuous layer of adhesive, a patterned layer of adhesive or any array of separate lines, spirals or spots of adhesive. In a preferred configuration, adhesive is applied to the second, or garment-facing, surface of the primary topsheet and then secured to an underlying acquisition layer or secondary topsheet of the absorbent article. This approach provides for a desired level of control over the ultimate location and disposition of the adhesive with respect to the extremities of the fluid passageways in the second surface of the primary topsheet. Alternatively, the adhesive may be applied to the wearer-facing surface of such the secondary layer such that it will directly and intimately contact the second or garment-facing surface of the polymeric film web once the components of the absorbent article are assembled.

The secondary layer may be a secondary topsheet, such as a fibrous nonwoven, or it may be an absorbent core component. For example, fibrous nonwoven materials suitable for use as a secondary layer in a topsheet system of the present invention include nonwoven webs formed of synthetic fibers (such as polypropylene, polyester, or polyethylene), natural fibers (such as wood, cotton, or rayon), or combinations of natural and synthetic fibers and blends thereof. Nonwoven webs may have fibers or filaments distributed randomly as in "air-laying" or certain "wet-laying" processes, or with a degree of orientation, as in certain "wet-laying" and "carding" processes.

Suitable nonwoven materials can be formed by various processes such as carding, spun-bonding, hydro-entangling, and other processes familiar to those knowledgeable in the art of nonwovens. A presently preferred fibrous nonwoven material is spunbond polypropylene, commercially available from Fiberweb of Simpsonville, S.C. Other preferred nonwoven webs include Fibrella 2200 NCB made by Suominen of Germany, and FG413MHB made by Walkisoft of Japan. An additional benefit of the use of adhesives having essentially no residual tack is realized during manufacture of topsheet systems that undergo mechanical deformation, i.e., compression forces, during processing. For example, after bonding the primary topsheet to a fibrous nonwoven secondary layer, the topsheet system may tend to be less flexible or less extensible due to the relative inflexibility or inextensibility of the bonded nonwoven. To render the nonwoven more flexible or extensible, the topsheet system may be processed by methods and apparatus used for elasticizing "zero strain" laminates by incremental stretching. An improved method for sequentially stretching a "zero strain" laminate web to impart elasticity thereto is disclosed in U.S. Pat. No. 5,143,679 issued to Weber et al. on Sept. 1, 1992 and hereby incorporated herein by reference. Further improvements are taught in U.S. Pat. Nos. 5,156,793 issued to Buell et al. on Oct. 20, 1992 and 5,167,897 issued to Weber et al. on Dec. 1, 1992, both of which are hereby incorporated by reference herein.

### ABSORBENT ARTICLE

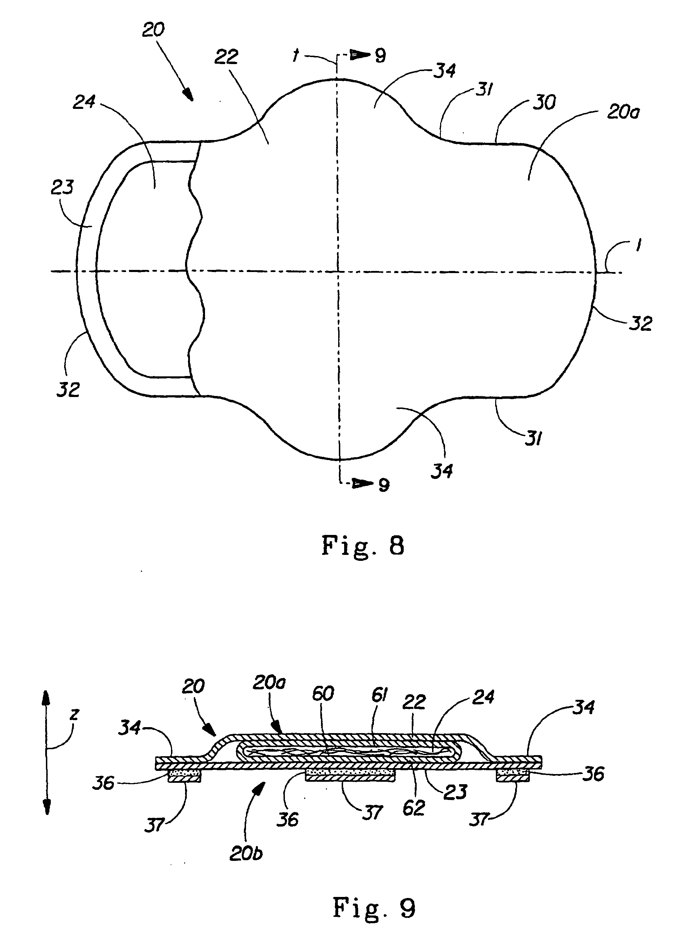

A representative embodiment of a topsheet system of the present invention utilized in a disposable absorbent article in the form of a catamenial pad, sanitary napkin 20, is shown in FIG. 8. As used herein, the term "sanitary napkin" refers to an absorbent article which is worn by females adjacent to the pudendal region, generally external to the urogenital region, and which is intended to absorb and contain menstrual fluids and other vaginal discharges from the wearer's body (e.g., blood, menses, and urine). Interlabial devices which reside partially within and partially external of the wearer's vestibule are also within the scope of this invention. As used herein, the term "pudendal" refers to the externally visible female genitalia. It should be understood, however, that the topsheet system of the present invention is also applicable to other absorbent articles such as panty liners, incontinent briefs, training pants, diapers, and the like. It should be understood, however, that the present invention is not limited to the particular type or configuration of sanitary napkin shown in FIG. 8, but is illustrated herein as a representative, nonlimiting example.

The sanitary napkin **20** has two surfaces, a wearer-contacting surface or body-contacting surface or "body surface" **20a** and a garment surface **20b.** The sanitary napkin **20** is shown in FIG. 8 as viewed from its body surface **20a.** The body surface **20a** is intended to be worn adjacent to the body of the wearer. The garment surface **20b** of the sanitary napkin **20** is on the opposite side and is intended to be placed adjacent to the wearer's undergarments when the sanitary napkin **20** is worn.

The sanitary napkin **20** has two centerlines, a longitudinal centerline 'l" and a transverse centerline "t". The term "longitudinal", as used herein, refers to a line, axis or direction in the plane of the sanitary napkin **20** that is generally aligned with (e.g., approximately parallel to) a vertical plane which bisects a standing wearer into left and right body halves when the sanitary napkin **20** is worn. The terms "transverse" or "lateral" as used herein, are interchangeable, and refer to a line, axis or direction which lies within the plane of the sanitary napkin **20** that is generally perpendicular to the longitudinal direction.

FIG. 8 is a top plan view of the sanitary napkin **20** of the present invention in its flat-out state with portions of the structure being cut-away to more clearly show the construction of the sanitary napkin **20** and with the portion which faces or contracts the wearer **20a,** oriented towards the viewer. As shown in FIG. 7, the sanitary napkin **20** preferably comprises a liquid pervious primary topsheet **22** (in a preferred embodiment, apertured, formed film primary topsheet **200),** a liquid impervious backsheet **23** joined with the topsheet **22,** and an absorbent core **24** positioned between the topsheet **22** and the backsheet **23.**

FIG. 8 also shows that the sanitary napkin **20** has a periphery **30** which is defined by the outer edges of the sanitary napkin **20** in which the longitudinal edges (or "side edges") are designated **31** and the end edges or "ends") are designated **32.**

Sanitary napkin **20** preferably includes optional side flaps or "wings" **34** that are folded around the crotch portion of the wearer's panties. The side flaps **34** can serve a number of purposes, including, but not limited to protecting the wearer's panties from soiling and keeping the sanitary napkin secured to the wearer's panties.

FIG. 9 is a cross-sectional view of the sanitary napkin **20** taken along section line 9-9 of FIG. 8. As shown, primary topsheet **22,** which can be apertured formed film topsheet **205,** as shown in FIG. 5, is adhered to subjacent layer **61,** which is shown as a nonwoven overwrap for absorbent core **24.** Alternatively, subjacent layer **61** can be nonwoven secondary layer **215** as shown in FIG. 5. Subjacent layer **61** may also comprise a scrim layer, tissue layer, or other disposable, fluid pervious layers, formed as a separate layer, or as an overwrap, as shown. Adhesive **220** is disposed between topsheet **22** and subjacent layer **61,** and can be an adhesive having essentially no residual tack **221** as shown in FIG. 5.

As can be seen in FIG. 9, the sanitary napkin **20** preferably includes an adhesive fastening means **36** for attaching the sanitary napkin **20** to the undergarment of the wearer. Removable release liners **37** cover the adhesive fastening means **36** to keep the adhesive from sticking to a surface other than the crotch portion of the undergarment prior to use.

In addition to having a longitudinal direction and a transverse direction, the sanitary napkin **20** also has a "z" direction or axis, which is the direction proceeding down through the topsheet **22** and into whatever fluid storage core **24** that may be provided. The objective is to provide a continuous path between the topsheet **22** and underlying layer or layers of the articles herein, such that fluid is eventually drawn in the "z" direction and away from the topsheet of the article into its ultimate storage layer. In a preferred embodiment the continuous path will have a gradient of increasing capillary attraction which facilitates fluid flow down into the storage medium.

In addition to the topsheet system. disclosed herein, specific disclosure of other individual components of the sanitary napkin, such as absorbent core materials, backsheets, and optional features, are disclosed in commonly assigned U.S. Pat. No. 5,342,334 issued August 30, 1994 to Thompson et al., hereby incorporated herein by reference.

It is believed that the description contained herein will enable one skilled in the art to practice the present invention in many and varied forms. Nonetheless, the following exemplary embodiment and analytical method is set forth for the purpose of illustrating the beneficial elastic reliability of a particularly preferred compression resistant web of the present invention.

### TEST METHOD

Residual tackiness of adhesives was determined by applying measured amounts of hot melt adhesive to sample substrates and allowing the adhesive to cool to room temperature before bonding the two materials together. The average peel force to separate the two substrates was quantified, to give the results in Table 1 above.

One of the pair of substrate materials was cut into 8" x 2.5" strips, adhered to a backing plate and conditioned at 73 degrees F and 50% RH for at least two hours prior to testing. After conditioning, glue was sprayed in a spiral pattern using a Nordson glue module at a basis weight of about 5.0 +/- 0.5 gsm on the outwardly facing side of the plate-mounted substrate. The single piece nozzle used for glue application had an orifice diameter of 0.012 inches. The width of the spiral was 1.7 cm, which is the distance between centers of two adjacent spirals. A sheet of release paper was promptly applied over the glue. After curing for at least 3 days the substrates were bonded together in pairs to form the test samples shown in Table 1 above.

Bonding was accomplished by bringing the plate-mounted substrate having glue applied into face to face contact with the second substrate after removal of the release paper. The second substrate was 2.5 inches wide, but significantly longer than the first, plate-mounted substrate. The extra length (i.e., greater than 8 inches) of the second substrate, which was necessary to accommodate the physical set up of the tensile tester as described below, extended beyond the end of the first substrate. Care was taken to ensure uniform bonding, i.e., no wrinkles, bubbles, etc., by placing each sample under uniform, full coverage pressure for 30 seconds. As shown in Table 1, test samples were measured under two conditions: 0.375 psi, and 1.25 psi. The pressure was obtained by placing the samples in a flat configuration under a suitable weight.

The weight was removed, and each sample was tested for peel strength using an EME portable tensile/compression tester, Model No. 607. One end of the plate having the plate-mounted sample was secured in the upper jaw of the tensile tester. The unadhered end of the second sample was then bent down and secured by the other jaw of the tester. Excess slack in the second substrate was eliminated, and the substrates were peeled apart at an angle of 180 degrees at a crosshead speed of about 40 inches/minute. Peel force data was collected for at least 6 inches of the adhered length of the two samples during peeling. For each substrate/adhesive combination, ten tests were run, with the average peel force normalized to grams/inch reported in Table 1.

Each sample was tested at two different times, as shown below in Table 2 (not normalized to grams/inch). As can be seen, the data shown in Table 1 is taken from the data in Table 2 for the shorter of the two time periods. While a longer time period results generally in a lesser peel force, the difference is considered to be inconsequential to the present invention.

**Table 2 Peel Force at Different Aging Times**

| | **Age (Days)** | **Average Peel Force (grams)** | | | | | |
|---|---|---|---|---|---|---|---|
| **Adhesive** | | **@0.375 psi** | | | **@1.25 psi** | | |
| | | **AW:NW** | **PE:PE** | **Cotton:PE** | **AW:NW** | **PE:PE** | **Cotton:PE** |
| Ato-Findley H2031 | 5 | 75±6 | 183±35 | 78±5 | 134±15 | 280±38 | 161±24 |
| | 14 | 64±6 | 106±14 | 66±7 | 122±8 | 187±23 | 149±12 |
| Ato-Findley HX4111 | 4 | 38±5 | 143±19 | 53±10 | 53±7 | 276±38 | 92±15 |
| | 7 | 36±5 | 150±23 | 52±9 | 53±7 | 221±18 | 92±9 |
| Fuller HL-1472-X ZP | 5 | 14±2 | 9±2 | 59±5 | 22±4 | 19±7 | 144±18 |
| | 14 | 11±1 | 6±2 | 56±4 | 11±3 | 18±6 | 114±18 |
| Ato-Findley H2600 | 2 | 10±1 | 4±1 | 38±7 | 17±3 | 16±6 | 148±27 |
| | 14 | 7±1 | 2±1 | 18±3 | 13±2 | 4±1 | 56±11 |
| National Starch 34-5643S | 5 | 0 | 0 | 2±1 | 0 | 0 | 6±2 |
| | 14 | 0 | 0 | 0 | 0 | 0 | 7±1 |
| Fuller HL-1628-B | 3 | 0 | 0 | 4±1 | 0 | 0 | 12±2 |
| | 14 | 0 | 0 | 3±1 | 0 | 0 | 11±2 |

## Claims

1. An absorbent article having a topsheet system, said topsheet system comprising:
(a) a primary topsheet comprising a first surface and a second surface generally parallel to and spaced apart from said first surface, a plurality of fluid passageways extending between said first surface and said second surface to place said first surface and said second surface in fluid communication with one another; and
(b) a secondary layer subjacent to and bonded to said second surface of said primary topsheet at predetermined portions by an adhesive **characterized in that** said adhesive is a hot melt adhesive exhibiting a residual tack of less than 16 grams per inch peel force, thus having essentially no residual tack, such that after joining, subsequent contact between said primary topsheet and said secondary layer at areas other than said predetermined portions does not result in additional bonding between said primary topsheet and said secondary layer.

2. The absorbent article of Claim 1, wherein said primary topsheet comprises an apertured formed polymeric film.

3. The absorbent article of Claim 2, wherein said primary topsheet further comprises a plurality of microapertures forming volcano-shaped surface aberrations.

4. The absorbent article of any of the preceding claims, wherein said secondary layer comprises a nonwoven web.

5. The absorbent article of any of the preceding claims, wherein said primary topsheet comprises a nonwoven web.

6. The absorbent article of any of the preceding claims, wherein said adhesive comprises a hot melt thermoplastic adhesive.

7. The absorbent article of Claim 1, wherein said adhesive exhibits a residual tack of less than 6 grams per inch peel force.

8. The absorbent article of Claim 1, wherein said adhesive exhibits a residual tack of about 0 grams per inch peel force.

9. The absorbent article of any of the preceding claims, wherein said absorbent article comprises a catamenial pad.

10. The absorbent article of any of the preceding claims, wherein said absorbent article comprises a sanitary napkin.

## Patentansprüche

1. Absorptionsartikel mit einem Oberschichtsystem, wobei das Oberschichtsystem Folgendes umfasst:
(a) eine primäre Oberschicht, umfassend eine erste Oberfläche und eine zweite Oberfläche, die zu der ersten Oberfläche generell parallel und davon beabstandet ist, mehrere Flüssigkeitskanäle, die zwischen der ersten Oberfläche und der zweiten Oberfläche verlaufen, um die erste Oberfläche und die zweite Oberfläche miteinander in Flüssigkeitsverbindung zu bringen, und
(b) eine sekundäre Schicht, die unter der zweiten Oberfläche der primären Oberschicht liegt und an vorgegebenen Abschnitten mittels eines Klebstoffs damit verbunden ist, **dadurch gekennzeichnet, dass** der Klebstoff ein Schmelzkleber ist, der eine Restklebrigkeit von weniger als 0,06 N/cm (16 Gramm pro Zoll) Schälkraft aufweist und somit im Wesentlichen keine Restklebrigkeit aufweist, so dass nach dem Verbinden ein nachfolgender Kontakt zwischen der primären Oberschicht und der sekundären Schicht in anderen Bereichen als den vorgegebenen Abschnitten nicht zu zusätzlicher Bindung zwischen der primären Oberschicht und der sekundären Schicht führt.

2. Absorptionsartikel nach Anspruch 1, wobei die primäre Oberschicht eine mit Öffnungen versehene geformte Polymerfolie umfasst.

3. Absorptionsartikel nach Anspruch 2, wobei die primäre Oberschicht ferner mehrere Mikroöffnungen umfasst, die vulkanförmige Oberflächenaberrationen bilden.

4. Absorptionsartikel nach einem der vorstehenden Ansprüche, wobei die sekundäre Schicht eine Vliesbahn umfasst.

5. Absorptionsartikel nach einem der vorstehenden Ansprüche, wobei die primäre Oberschicht eine Vliesbahn umfasst.

6. Absorptionsartikel nach einem der vorstehenden Ansprüche, wobei der Klebstoff einen thermoplastischen Schmelzkleber umfasst.

7. Absorptionsartikel nach Anspruch 1, wobei der Klebstoff eine Restklebrigkeit von weniger als 0,02 N/cm (6 Gramm pro Inch) Schälkraft aufweist.

8. Absorptionsartikel nach Anspruch 1, wobei der Klebstoff eine Restklebrigkeit von ungefähr 0 N/cm (0 Gramm pro Inch) Schälkraft aufweist.

9. Absorptionsartikel nach einem der vorstehenden Ansprüche, wobei der Absorptionsartikel eine Menstruationsbinde umfasst.

10. Absorptionsartikel nach einem der vorstehenden Ansprüche, wobei der Absorptionsartikel eine Damenbinde umfasst.

## Revendications

1. Article absorbant ayant un système de feuille de dessus, ledit système de feuille de dessus comprenant :
(a) une feuille de dessus primaire comprenant une première surface et une deuxième surface généralement parallèle à et espacée de ladite première surface, une pluralité de passages de fluide s'étendant entre ladite première surface et ladite deuxième surface pour placer ladite première surface et ladite deuxième surface en communication du point de vue des fluides l'une avec l'autre ; et
(b) une couche secondaire sous-jacente et liée à ladite deuxième surface de ladite feuille de dessus primaire au niveau de parties prédéterminées par un adhésif, **caractérisé en ce que** ledit adhésif est un adhésif thermofusible présentant une adhésivité résiduelle inférieure à 0,06 N/cm (16 grammes par pouce) de force de pelage, n'ayant ainsi pratiquement aucune adhésivité résiduelle, de telle sorte qu'après raccordement, un contact ultérieur entre ladite feuille de dessus primaire et ladite couche secondaire au niveau de zones autres que lesdites parties prédéterminées n'entraîne pas de liaison supplémentaire entre ladite feuille de dessus primaire et ladite couche secondaire.

2. Article absorbant selon la revendication 1, dans lequel ladite feuille de dessus primaire comprend un film polymère formé perforé.

3. Article absorbant selon la revendication 2, dans lequel ladite feuille de dessus primaire comprend en outre une pluralité de microperforations formant des irrégularités de surface en forme de volcan.

4. Article absorbant selon l'une quelconque des revendications précédentes, dans lequel ladite couche secondaire comprend une nappe non tissée.

5. Article absorbant selon l'une quelconque des revendications précédentes, dans lequel ladite feuille de dessus primaire comprend une nappe non tissée.

6. Article absorbant selon l'une quelconque des revendications précédentes, dans lequel ledit adhésif comprend un adhésif thermoplastique thermofusible.

7. Article absorbant selon la revendication 1, dans lequel ledit adhésif présente une adhésivité résiduelle inférieure à 0,02 N/cm (6 grammes par pouce) de force de pelage.

8. Article absorbant selon la revendication 1, dans lequel ledit adhésif présente une adhésivité résiduelle d'environ 0 N/cm (0 gramme par pouce) de force de pelage.

9. Article absorbant selon l'une quelconque des revendications précédentes, où ledit article absorbant comprend un tampon cataménial.

10. Article absorbant selon l'une quelconque des revendications précédentes, où ledit article absorbant comprend une serviette hygiénique.
